Europäisches Patentamt

European Patent Office

Office européen des brevets

(11) Publication number: **0 303 483**
**A2**

# EUROPEAN PATENT APPLICATION

(21) Application number: **88307438.7**

(22) Date of filing: **11.08.88**

(51) Int. Cl.⁴: **C 12 P 21/00**
**A 61 K 37/02**
**//(C12P21/00,C12R1:91)**

(30) Priority: **14.08.87 US 85176**

(43) Date of publication of application:
**15.02.89 Bulletin 89/07**

(84) Designated Contracting States:
**AT BE CH DE ES FR GB GR IT LI LU NL SE**

(71) Applicant: **THE UNIVERSITY OF BRITISH COLUMBIA**
**2075 Wesbrook Mall**
**Vancouver British Columbia V6T 1W5 (CA)**

(72) Inventor: **Teh, Hung-Sia**
**2015 West 47th Avenue**
**Vancouver British Columbia (CA)**

**McMaster, William**
**4654 West 8th Avenue**
**Vancouver British Columbia (CA)**

(74) Representative: **Goldin, Douglas Michael et al**
**J.A. KEMP & CO. 14, South Square Gray's Inn**
**London WC1R 5EU (GB)**

(54) Novel lymphokine and compositions thereof.

(57) A soluble factor of approximately 27kDa has been identified which, in the absence of antigen, cooperates with interleukin-2 to induce proliferation of precursor cytotoxic T-cells. Compositions and methods useful in inducing cytotoxic T-cell activity are described.

EP 0 303 483 A2

**Description**

## NOVEL LYMPHOKINE AND COMPOSITIONS THEREOF

FIELD OF THE INVENTION

This invention relates to soluble factors involved in the cell-mediated immune response.

BACKGROUND OF THE INVENTION

A better understanding of the mechanism by which the immune system responds when challenged by antigen would assist researchers in developing therapies for counteracting deficiencies in the immune response. Current evidence supports the concept that a cascade of interactions, mediated in part by soluble factors known as lymphokines, results ultimately in the triggering of cytotoxic T-cells, a sub-class of lymphocytes which destroy infected, foreign or malignant cells by lysing them.

REFERENCE TO THE PRIOR ART

The mechanism by which cytotoxic T-cells are activated has been the subject of many recent publications. Early evidence has indicated that interleukin-2 has a role in activating cytotoxic T-cells by interacting with precursor cytotoxic T-cells. According to Erard et al (J. Immunol, 134, 1985) a combination of exposure of precursor cytotoxic T-cells to antigen and then interaction of those cells with interleukin-2 per se causes cytotoxic T-cell proliferation. These results support well the concept that cytotoxic T-cell precursors respond to stimulation by antigen by expressing interleukin-2 receptors. Simultaneous antigen exposure of a sub-class of lymphocytes known as T4 or T-helper calls induces the T-helper cells to secrete interleukin-2 which then interacts with the antigen-sensitized cytotoxic T-cells to induce their proliferation. Resulting cytotoxic T-cells are specific for the antigen which has sensitized them, an affinity which facilitates a specific immune response.

While the evidence provided by Erard et al, 1985 supra indicates that only antigen and interleukin-2 are required to induce cytotoxic T-cell proliferation, results of an experiment of similar design reported by Takai et al (J. Immunol, 137, 1986) indicate that two additional factors are required; gamma-interferon and 31KD soluble factor termed cytotoxic T-cell differentiation factor (CDF). Despite these apparently contradictory results, both investigators have confirmed that a combination of sensitization by antigen and interaction with lymphokine(s) can induce cytotoxic T-cells to proliferate into a clonal population specific for the sensitizing antigen.

Results suggesting that cytotoxic T-cell proliferation may be induced by an antigen-independent pathway were reported by Teh et al in J. Immunol, 131, 1983. These researchers found that when extracellular medium, in which an interleukin-2 producing murine lymphoma line was cultured, was added to a T-cell enriched population, cytotoxic T-cells proliferated which were non-specific for antigen bearing cells. The polyclonally activated T-cells were cytotoxic for a broad spectrum of allogeneic target cells. Analysis of the components within the extracellular medium to which the precursor cytotoxic T-cells had been exposed suggested strongly that interleukin-2, without antigen, was responsible for inducing cytotoxic T-cell proliferation.

An ability to induce cytotoxic T-cell proliferation without the need for stimulation by antigen is of particular interest. Therapies designed to restore immune function can be of limited value if they depend for their success on introducing antigen to a system which is being harmed by that antigen or where the precursor cell has lost receptivity to antigen. It would be much more desirable to identify and develop an alternative therapy using naturally produced factors which act to induce the immune response, thereby to circumvent any malfunction in the intercellular communication which leads ultimately, in the cell-mediated response, to cytotoxic T-cell activation.

It is, accordingly, one object of the present invention to identify soluble factors which cooperate, in the absence of antigen, to activate cytotoxic T-cells.

It is a further object of the present invention to provide a method for the therapeutic use of the soluble factors so identified.

SUMMARY OF THE INVENTION

The present inventors have found that a soluble factor to be described herein is capable of co-acting with interleukin-2, in the absence of antigen, to induce the proliferation of precursor cytotoxic T-cells. As assessed by column separation on a G-100 chromatographic column, the novel soluble factor of the present invention has an apparent molecular weight of 27kDa. Accordingly, for convenience herein the soluble factor of the present invention is referred to as the 27kDa factor. It will be appreciated from an understanding of the following description, however, that the molecular weight attributed to the novel factor of the present invention is an approximate one. The 27kDa factor is more appropriately defined with reference to its biological function and its original source.

The 27kDa factor was recovered from the extracellular medium in which phorbol-12-myristate-13-acetate (PMA)-stimulated murine lymphoma line EL4.IL-2 was cultured, using techniques very similar to those described by Teh et al, 1983 supra, the entire contents of which are incorporated herein by reference. This cell line EL4.IL-2 is on publicly accessible deposit with the American Type Culture Collection, Rockville, Maryland, U.S.A. where it has been accorded accession number ATCC® TIB 181. Although this cell line producs interleukin-2 and other lymphokines as well as the 27kDa factor, the 27kDa factor has been substantially separated from interleukin-2 in order to assess the functions of these two soluble factors, and other soluble factors, separately.

Thus, in accordance with one aspect of the present invention, there is provided a novel soluble factor, which is recoverable from the extracellular medium in which EL4.IL-2 (ATCC TIB 181) cells have been cultured in the presence of phorbol-12-myristate-13-acetate and which is capable when combined with interleukin-2 of inducing the proliferation of non-specific cytotoxic T-cells from precursor cytotoxic T-cells.

Although the exact mechanism by which the 27kDA factor participates in cytotoxic T-cell activation is not known evidence suggests that the 27kDa factor induces precursor cytotoxic T-cells to express the interleukin-2 receptor, eliciting much the same result as does antigen stimulation although probably by different interaction. For the purposes of this description, the result of communication between the 27kDa factor and the precursor cytotoxic T-cell is said to be a "sensitized" precursor cytotoxic T-cell, which is responsive to the proliferation-inducing effect of interleukin-2.

Accordingly, a further aspect of the present invention resides in a composition of matter comprising a precursor cytotoxic T-cell sensitizing amount of the novel soluble factor of the present invention as defined hereinabove and a cytotoxic T-cell-proliferation-inducing amount of a compound having interleuking-2 activity. Preferably, the composition of the present invention is substantially free of antigen or mitogen. Also, preferably, the composition of the present invention is substantially free of soluble factors other than the 27kDa factor and interleukin-2.

A particularly valuable aspect of the present invention resides in the use of the composition defined above in activating cytotoxic

T-cells, for example, in serum obtained from patients whose immune response has been interrupted at some point in the cytotoxic T-cell activation pathway. For example, the composition comprising the 27kDa factor and interleukin-2 may be used in vivo, being administered in suitable form such as by intravenous or intramuscular injection or may be used in vitro to treat a serum sample comprising precursor cytotoxic T-cells extracted from a patient. Treated cells may then be re-introduced by intravenous injection.

Accordingly, a further aspect of the present invention comprises a method for inducing cytotoxic T-cell proliferation which comprises treating a population of precursor cytotoxic T-cells with the composition of the present invention as defined hereinabove.

As the terms are used herein, "precursor cytotoxic T-cells" are progenitor lymphocyte cells which when activated by the composition of the present invention proliferate as antigen-non-specific cytotoxic T-cells; cytotoxic T-cells are lymphocytes recognizable by their antigenic markers as follows: Thy-1$^+$, Lyt-2$^+$ (CD8$^+$), L3T4$^-$ (CD4$^-$).

## DETAILED DESCRIPTION OF THE PREFERRED EMBODIMENTS

The 27kDa factor is produced by the murine lymphoma line EL4.IL-2 (ATCC TIB 181) when exposed to phorbol-12-myristate-13- acetate (PMA). The cell line EL4.IL-2 is a sub-line of EL4 (ATCC$^R$ TIB 39) and produces IL-2 which is biochemically and biologically indistiguishable from normal spleen cell-derived IL-2). Culture medium in which these PMA-stimulated cells have been grown is therefore a valuable source from which to extract the 27kDa factor, using any technique allowing a separation of components according to molecular size and/or charge such as chromatography and electrophoresis. Alternatively, monoclonal antibodies specific for the 27kDa factor may, when developed, be used to simplify the purification procss, e.g. using affinity chromatography.

It will be appreciated, given the current state of biotechnology, that alternative 27kDa factor sources may be developed including microbial expression, hybridoma expression, etc. Provided that these alternative production strategies are able to produce a molecule having biochemical properties and biological activity equivalent to the 27kDa factor in the terms described herein, the engineered molecule will be suitable for use in combination with interleukin-2 according to the method of the present invention.

Interleukin-2 useful in the present invention is now well known in the art. It is produced by T-cells stimulated by lectin, allotype antigen, mitogen, etc. and may be recovered therefrom using procedures and cell lines described, for example in EP132,359 (published January 30, 1985), EP92,163 (published October 26,1983) U.S. 4,401,756; U.S. 4,411,992; 4,448,879; 4,473,642 and 4,623,622. Alternatively, the interleukin-2 may be produced by microbial expression systems such as in the E. coli system described in WO 86/01221; EP 119,621 (published September 26, 1984); EP 136,489 (published April 10, 1985) and EP 172,619 (published February 26, 1985). Moreover, interleukin-2 is available commercially from Amgen, California, U.S.A.

Various natural sources of interleukin-2 such as murine and human T-cells are available as are various derivatives of natural interleukin-2 including a mutein in which the amino acid in position 125 has been changed from cysteine to serine (see U.S. 4,518,584), a mutein in which the initial alanine residue is missing and a truncated version in which the last four amino acids are absent. Provided that the compound serves the biological function of interleukin-2, presumably that of binding to its receptor on sensitized precursor cytotoxic T-cells and thereby inducing cytotoxic T-cell proliferation, derivatives of interleukin-2 and native interleukin-2 obtained from various sources may be used together with the 27kDa factor in the composition of the present invention. Selection of the preferred interleukin-2 compound will be dictated by economic considerations and by a desire to provide for superior biological efficacy in the composition of the present invention.

As described above, the composition of the present invention comprises a sensitizing amount of the 27kDa factor and a proliferation -indlucing amount of a compound having interleukin-2 activity. Such a composition may be formulated for parenteral administation using, for example, the pharmaceutically acceptable carrier described in U.S. 4,604,377. Briefly, the composition described in U.S. 4,604,377 is a sterile, stable, lyophilized formulation of selectivity oxidized, microbially-produced recombinant interleukin-2. The interleukin is admixed with a water-soluble carrier such as mannitol and a sufficient amount of surfactant such as sodium dodecyl sulfate to ensure solubility of the interleukin in water. Such a composition can be reconstituted in aqueous medium for injection parenterally and is accordingly well suited as a means for introducing the composition of the present invention. It will be necessary simply to add a sufficient amount of the 27kDa factor to the composition described in U.S. 4,604,377 and to alter as necessary the surfactant concentration to ensure that the 27kDa is solubilized as well after aqueous reconstitution of the 27kDa and interleukin-2 composition. The entire contents of U.S. 4,604,377 are incorporated herein by reference.

Both the 27kDa factor and the interleukin-2 are moderately hydrophobic and will require some surfaction in aqueous medium. An alternative delivery system may be used, as outlined in GB 2,157,172 published October 23, 1985, to provide for sustained release of the active agents, using liposomes as delivery agents. For example, a hydrophilic solution of interleukin-2 and the 27kDa factor may be mixed with lipid in organic solvent, treated to remove liquid, residue taken up in buffered solution to form a suspension which is then subjected to ultrasonication to form liposomes. These liposomes containing desired relative amounts of interleukin-2 and 27kDa factor may then be selected, providing a valuable means for introducing the active agents to the target site, such as by injecting an aqueous solution of the liposomes.

Generally, methods useful in delivering interleukins will be useful also in delivering the composition of the present invention.

The relative amounts of the 27kDa factor and the interleukin-2 required to induce cytotoxic T-cell proliferation can be determined using protocols standard in clinical trials. Compositions containing in concentration from $10^{-8}$ to $10^{-13}$M 27kDa factor should be suitable for injection, preferably $10^{-10}$ to $10^{-12}$M. Thus, solutions containing interleukin-2 in concentrations as described in U.S. 4,604,377 may be supplemented with amounts of the 27kDa factor to produce compositions useful herein. It is possible, however, because these factors act synergistically to induce cytotoxic T-cell proliferation, to use marginally less IL-2 than described in U.S. 4,604,377.

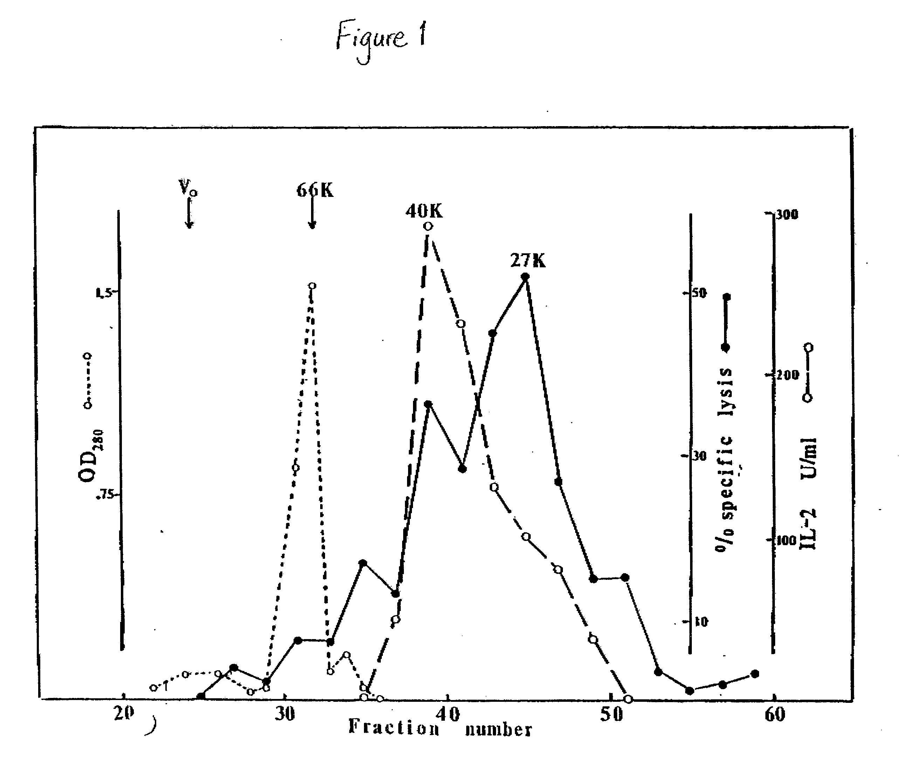

Certain aspects of the present invention are illustrated hereinafter by way of example only, with reference to the accompanying drawings in which Figure 1 illustrates the results of chromatographic fractionation of supernatant from EL4.IL-2 cells cultured in the presence of PMA.

Example 1 - Production of 27kDa Factor

The 27kDa factor was produced by stimulating EL4.IL-2 cells (ATCC TIB 181) with phorbol-12-myristate-13-acetate (PMA) (a product of Sigma Chemical Co., St. Louis, MO, U.S.A.) as described by Farrar et al in J. Immunol 1980 125:2555, incorporated herein by reference. Briefly, the EL4.IL-2 cells were cultured at $10^6$ cells/ml in RPMI 1640 medium (a product of Gibco Laboratories) supplemented with 5% bovine serum and 10 ng/ml PMA for 24 hours at 37°C. Supernatant obtained after pelletting cells is herein termed EL4.PMA and comprises, inter alia, the 27kDa factor of the present invention.

In assessing the EL4.PMA supernatant for lymphokine activity, certain techniques and materials common to the experimentation described in the following examples were used and are reproduced below for convenience:

Mice: as sources for various tissues and lymphocytes populations true-breeding mice bred in the animal unit of the Department of Microbiology were used and included C5BL/6 (B6); DBA/2 (D2); Balb/c; CBA/J (CBA) and (B6 x D2)F1 (BDF1).

Cultures and Assays: The culture medium used was Iscove's Modified Dulbecco's medium supplemented with 10% fetal bovine serum (Bocknek, Rexdale, Ontario), 5 x $10^{-5}$M 2-mercaptoethanol, 50 U/ml of Penicillin and 50 g/ml streptomycin. All cultures were incubated at 37°C in 5% $CO_2$ in humid air.

For CTL assays, cultures were set up by incubating the indicated number of spleen cells, lymph node cells or thymocytes with the appropriate lymphokine in conical bottomed microtiter plates (Flow Laboratories, #76-023-05) as previously described by Teh et al, 1983, supra. Five days later the cultures were assessed for cytotoxic activity in phytohemaglutinin (PHA-P)-mediated cytotoxicity assay as described by Bevan and Cohn, J. Immunol 1975 114:559. This assay detects CTL of all specificities. A total of 3 x $10^3$ $^{51}$Cr-labelled P815 (H-$2^d$) mastocytoma target cells (available from ATCC®) and 10 g/ml of PHA-P were added to each well. Spontaneous release during the 3.5 hr assay period was about 10% of the maximum releasable counts.

Proliferative responses were determined by the incorporation of $^3$H-thymidine into proliferation cells. Lymphocytes (3 x $10^4$/well) were incubated with the indicated lymphokine for 3 days in a volume of 0.20 ml in round bottomed microtiter trays. One Ci of $^3$H-thymidine was added to each well during the last 6 hr of culture.

## Monoclonal Antibodies

To identify the type of cells involved in the interactions under investigation labelled monoclonal antibodies were used which were specific for the antigenic marker representative of a particular cell type. For example, the antigenic marker known as Thy-1.2 is present on the surface of all T-cells and can be bound to Jlj antibody. Similarly, monoclonal antibody 7D4 against the IL-2 receptor will bind to this receptor. Monoclonal antibody GK1.5 is specific the L3T4 antigen present on the surface of all T-helper cells and ATCC T1B105 antibody is specific fo the mouse LyT2 antigen found on all cytotoxic cells. B cells hybridoma lines producing these monoclonal antibodies were obtained from the American Type Culture Collection. Two other monoclonal antibodies, one specific for the beta-chain of the T-cell receptor and another specific for interleukin-4 were also used in the assessment. Sources of these monoclonal antibodies include spent culture medium which had been concentrated five times or 50% $(NH_4)_2$ $SO_4$ precipitates of the relevant ascitic fluids.

The percentages of cells expressing molecules bound by the monoclonal antibodies were determined by incubating the test cells with a saturating amount of fluorescein isothiocyanate FITC conjugated second antibodies. FITC rabbit anti-mouse IgG and FITC-rabbit anti-rat IgM were used to detect monoclonal antibody binding. The FITC labelled cells were analyzed by flow cytometry using the fluorescence activated cell sorter FACS IV.

## Example 2 - Assessment of EL4.PMA Lymphokine Activity

EL4.PMA contains a number of lymphokines in addition to interleukin-2. These lymphokines include IL-3, IL-4, colony stimulating factor and a T cell cytotoxicity-inducing factor described by Mannel and Falk in "Cellular and Molecular Biology of Lymphokines", Academic Press, 1985, p. 93.

With the availability of recombinant interleukin-2 (rII-2) it can be demonstrated that the induction of cytotoxic T-cells by EL4.PMA is dependent on factors with other IL-2. When $5 \times 10^3$ speen cells from BDF1 mice were incubated with 5 to 40 U/ml rIL-2 for five days, no proliferative or cytotoxic responses were detected in these cultures as indicated in Table 1 below.

## Table 1

| BDF1 spleen cells | EL4.PMA[b] % (v/v) | rIL-2 U/ml | 7D4 ug/ml | $^3$H-TdR Incorporation CPM[c] | SE | % of lysis M[d] | SE |
|---|---|---|---|---|---|---|---|
| $5 \times 10^3$ | | | | $741 \pm 62$ | | $1.4 \pm 0.5$ | |
| $5 \times 10^3$ | 10 | | | $16,585 \pm 1275$ | | $72.0 \pm 2.9$ | |
| $5 \times 10^3$ | 5 | | | $3,251 \pm 589$ | | $27.3 \pm 3.1$ | |
| $5 \times 10^3$ | 2.5 | | | $1,113 \pm 233$ | | $5.1 \pm 1.6$ | |
| $5 \times 10^3$ | 1.25 | | | $830 \pm 89$ | | $2.6 \pm 0.8$ | |
| $5 \times 10^3$ | | 40 | | $1,035 \pm 655$ | | $0.9 \pm 0.6$ | |
| $5 \times 10^3$ | | 20 | | $2,925 \pm 1446$ | | $-0.6 \pm 0.7$ | |
| $5 \times 10^3$ | | 10 | | $1,401 \pm 880$ | | $2.4 \pm 0.7$ | |
| $5 \times 10^3$ | | 5 | | $507 \pm 86$ | | $0.0 \pm 0.8$ | |
| $5 \times 10^3$ | 10 | | 100 | n.d.[e] | | $14.9 \pm 8.1$ | |
| $5 \times 10^3$ | 10 | | 30 | n.d.[e] | | $45.8 \pm 4.0$ | |
| $5 \times 10^3$ | 10 | | 10 | n.d.[e] | | $54.1 \pm 5.6$ | |

[a]BDF1 spleen cells were cultured with EL4.PMA or rIL-2. After 3 days the cultures were assayed for proliferative responses. Cytotoxic responses were assayed after 5 days in a PHA-P-mediated assay. Details for these assays are described above.

[b]This batch of EL4.PMA at 100% concentration contained 400 U/ml of IL-2.

[c]Mean of 4 cultures.

[d]Mean of 6 cultures.

[e]not determined.

By contrast, when $5 \times 10^3$ BDF1 spleens were incubated with 1.25 to 10% (v/v) EL4.PMA, which contained 5 to 40 U/ml of IL-2, both a proliferative response as measured by incorporation of $^3$H thymidine into these cells ($^3$H-TdR Incorporation) and a cytotoxic response as measured by release by radioactivity chromium from target cells (% specific lysis) were induced. Moreover, the magnitude of the proliferative and cytotoxic responses was proportional to the concentration of EL4.PMA included in the cultures. The EL4.PMA-induced cytotoxic response was dependent on IL-2 since this response was inhibited when cells were exposed to the IL-2 receptor antibody (7D4) which prevents IL-2 binding on activated T cells.

This finding suggests that EL4. PMA contained factors in addition to IL-2 which can synergize with IL-2 in activating cytotoxic T-cells.

## Example 3 - Effect of IL-2 per se on induction

Recombinant IL-2 by itself could also induce a cytotoxic response when BDF1 spleen cells were cultured at $2 \times 10^4$ or $4 \times 10^4$/well as shown below in Table 2.

Table 2. Effect of varying cell density on proliferative and cytotoxic responses to EL4.PMA and IL-2

| BDF1 spleen cells | EL4.PMA % (v/v) | rIL-2 U/ml | CPM SE | % specific lysis |
|---|---|---|---|---|
| $4 \times 10^4$ | | | $1,973 \pm 136$ | $2.4 \pm 1.8$ |
| $2 \times 10^4$ | | | $886 \pm 58$ | $1.2 \pm 1/8$ |
| $1 \times 10^4$ | | | $567 \pm 48$ | $0.0 \pm 1.5$ |
| $5 \times 10^4$ | | | $1,262 \pm 786$ | $1.7 \pm 1.1$ |
| $2.5 \times 10^3$ | | | $1,349 \pm 457$ | $1.0 \pm 1.1$ |
| $4 \times 10^4$ | 10 | | $49,238 \pm 811$ | $84.1 \pm 5.4$ |
| $2 \times 10^4$ | 10 | | $28,126 \pm 592$ | $81.7 \pm 4.7$ |
| $1 \times 10^4$ | 10 | | $17,867 \pm 725$ | $81.7 \pm 4.3$ |
| $5 \times 10^3$ | 10 | | $9,196 \pm 128$ | $77.0 \pm 8.9$ |
| $2.5 \times 10^3$ | 10 | | $5,384 \pm 536$ | $54.8 \pm 8.9$ |
| $4 \times 10^4$ | | 20 | $5,015 \pm 450$ | $58.0 \pm 8.1$ |
| $2 \times 10^4$ | | 20 | $1,107 \pm 67$ | $31.2 \pm 8.0$ |
| $1 \times 10^4$ | | 20 | $847 \pm 321$ | $8.0 \pm 3.5$ |
| $5 \times 10^3$ | | 20 | $975 \pm 41$ | $6.0 \pm 3.1$ |
| $2.5 \times 10^3$ | | 20 | $499 \pm 38$ | $0.0 \pm 0.7$ |

Culture and assay conditions were as described in Table 1.

By contrast, EL4.PMA could induce strong proliferative and cytotoxic responses at all cell densities tested ($2.5 \times 10^3$ to $4 \times 10^4$). It is clear from this study that the cell responsive to IL-2 alone is present in low frequency in normal spleen cells and does not contribute to the proliferative and cytotoxic response in low density cultures. EL4.PMA apparently contains a factor(s) that can render non-IL-2 responsive cells responsive to IL-2. In this assay system, PMA and rIL-2 had only a minimal effect in inducing CTL (see Table 3 below). Thus, in the presence of excess rIL-2, a weak CTL response was induced when PMA was added at 0.1 or 0.3 ng/ml (lines 7 and 8). This response was considerably weaker than that observed with various concentrations of EL4.PMA (lines 1 to 4). Therefore, the effects observed with EL4.PMA could not be accounted for by the presence of residual PMA and IL-2 in EL4.PMA.

## Table 3

| BDF1 spleen cells | EL4.PMA % (v/v) | rIL-2 U/ml | PMA ng/ml | % specific lysis (M    SE) |
|---|---|---|---|---|
| $5 \times 10^3$ | 10 | | | 68.6 ± 2.0 |
| $5 \times 10^3$ | 5 | | | 47.7 ± 3.3 |
| $5 \times 10^3$ | 2.5 | | | 25.5 ± 4.4 |
| $5 \times 10^3$ | 1.25 | | | 11.1 ± 5.8 |
| $5 \times 10^3$ | | 20 | | -0.6 ± 1.5 |
| $5 \times 10^3$ | | 20 | 0.03 | 3.5 ± 1.5 |
| $5 \times 10^3$ | | 20 | 0.1 | 15.7 ± 5.1 |
| $5 \times 10^3$ | | 20 | 0.3 | 14.8 ± 3.3 |
| $5 \times 10^3$ | | 20 | 1.0 | 2.8 ± 1.3 |
| $5 \times 10^3$ | | 20 | 3.0 | 4.3 ± 1.4 |
| $5 \times 10^3$ | | 20 | 10.0 | -0.4 ± 0.9 |
| $5 \times 10^3$ | | | 0.1 | -2.2 ± 1.4 |
| $5 \times 10^3$ | | | 0.3 | -3.2 ± 1.7 |
| $5 \times 10^3$ | | | 1.0 | -2.5 ± 0.9 |
| $5 \times 10^3$ | | | 3.0 | -1.0 ± 1.8 |
| $5 \times 10^3$ | | | 10.0 | -0.9 ± 1.6 |

Culture and assay conditions were as described in Table 1.

Example 4 - Identifying the EL4.PMA Target Cell

The target cell for EL4.PMA was determined by passing B6 lymph node (LN) cells over a nylon wool column to enrich for T cells (36). The nylon wool nonadherent cells were depleted of Thy-1+, Lyt2+ or L3T4+ cells by treatment with mAbs directed to these cell surface molecules and complement. The treated cells were then cultured with EL4.PMA. It can be seen from Table 4 that the cell that responds to EL4.PMA is a nylon wool nonadherent, Thy-1+, Lyt2+ L3T4- cell.

## Table 4

| B6 LN cells | Treatment | EL4.PMA 10% (v/v) | $^3$H-TdR Incorporation (CPM    S.E.) | % specific lysis (M    SE) |
|---|---|---|---|---|
| 3 x 10$^4$ | Untreated | - | 282 $\pm$ 123 | -1.3 $\pm$ 1.2 |
| 1 x 10$^4$ | | - | 710 $\pm$ 117 | -0.7 $\pm$ 1.2 |
| 3 x 10$^3$ | | - | 1,164 $\pm$ 120 | -0.2 $\pm$ 2.2 |
| 3 x 10$^4$ | Untreated | + | 17,426 $\pm$ 1311 | 54.0 $\pm$ 6.6 |
| 1 x 10$^4$ | | + | 5,021 $\pm$ 579 | 34.9 $\pm$ 9.6 |
| 3 x 10$^3$ | | - | 2,272 $\pm$ 203 | 3.0 $\pm$ 2.1 |
| 3 x 10$^4$ | Untreated | + | 14,065 $\pm$ 5746 | 39.8 $\pm$ 4.3 |
| 1 x 10$^4$ | | + | 3,236 $\pm$ 578 | 12.2 $\pm$ 3.9 |
| 3 x 10$^3$ | | + | 2,379 $\pm$ 459 | 0.4 $\pm$ 1.0 |
| 3 x 10$^4$ | NW, -Thy-1 + C | + | 2,347 $\pm$ 1019 | -1.7 $\pm$ 1.2 |
| 1 x 10$^4$ | | + | 3,309 $\pm$ 1616 | -2.1 $\pm$ 0.9 |
| 3 x 10$^3$ | | + | 1,914 $\pm$ 1255 | -1.6 $\pm$ 1.6 |
| 3 x 10$^4$ | NW, Lyt2 + C | + | 2,194 $\pm$ 54 | -1.2 $\pm$ 1.9 |
| 1 x 10$^4$ | | + | 2,033 $\pm$ 706 | -1.1 $\pm$ 1.5 |
| 3 x 10$^3$ | | + | 1,080 $\pm$ 478 | 0.2 $\pm$ 0.4 |
| 3 x 10$^4$ | NW, L3T4 + C | + | 18,562 $\pm$ 978 | 35.1 $\pm$ 1.1 |
| 1 x 10$^4$ | | + | 5,827 $\pm$ 1050 | 5.0 $\pm$ 2.3 |
| 3 x 10$^3$ | | + | 1,923 $\pm$ 123 | -0.6 $\pm$ 1.3 |

Thus, removal of nylon wool adherent cells or L3T4$^+$ cells from B6 LN cells did not significantly reduce the proliferative or cytotoxic response (compare lines 4 to 6 with 7 to 9 and 16 to 18, respectively). However, both responses were completely abrogated by treating the nonadherent cells with either anti-Thy-1 or anti-Lyt-2 mAbs and complement (lines 10 to 15). Nylon wool nonadherent B6 LN cells that had been treated with the mAb to L3T4 and complement were cultured for 3 days in EL4.PMA. After culture for 3 days in EL4.PMA there was a nine-fold increase in cell number indicating that most of the cells had proliferated in EL4.PMA. The cells at the end of the 3-day culture period were checked for the expression of the Lyt2, L3T4, IL-2 receptor and T-cell receptor. Most of the cultured cells were Lyt2$^+$ (78%) and L3T4$^-$ (3%). A significant number of these cells expressed IL-2 receptors (32%). Similarly, a population of cells also expressed the chain determinant of the T-cell receptor.

Thus, EL4.PMA contains a factor(s) that can induce the expression of the IL-2 receptor on Thyl$^+$ Lyt2$^+$ L3T4$^-$ cells in normal lymph node cells. Cells expressing IL-2 receptors then proliferate and differentiate into CTL in the presence of IL-2. By contrast to spleen cells, B6 LN cells cultured with rIL-2 under the same conditions did not proliferate and did not give rise to cells expressing IL-2 receptor.

Example 5 - Polyclonal Activation by EL4.PMA

The preceding data suggest that most, if not all, cytotoxic T-cells (Lyt2$^+$ L3T4$^-$) cells were activated by EL4.PMA. To determine whether CTL-P of all antigenic specificities were activated by EL4.PMA, B6 spleen cells were cultured in EL4.PMA and the effector cells were assayed for cytotoxic activity against syngeneic B6 and allogeneic CBA, D2 and P815 target cells. Little or no cytotoxic effector cells were produced against syngeneic B6 (H-2$^b$) target cells. Approximately the same number of effector cells were generated against D2 (H-2$^d$) or CBA (H-2$^k$) target cells. CTL of all specificities were determined by assaying for cytolytic activity against P815 targets in the presence of PHA-P. The total number of cytotoxic effector cells produced was more than a hundred-fold greater than the number of effector cells against any one specific target cells. Thus, EL4.PMA appears to polyclonally activate CTL-P of all antigenic specificities in the apparent absence of mitogen or antigen stimulation.

Example 6 - Identification of 27kDa factor in EL4.PMA

The factor in EL4.PMA that synergized with rIL-2 in the activation of CTL-P eluted with an apparent molecular weight of 27kDa from a Sephadex G-100 column (Fig. 1). The IL-2 in EL4.PMA eluted with an apparent molecular weight of 40kDa on this column and was partially separated from the 27kDa factor. Since the CTL-inducing activity of EL4.PMA is associated with 27kDa factor this suggests that the effects of EL4.PMA are unlikely to be due to residual PMA in EL4.PMA. Previous studies using radiolabelled PMA have shown that although PMA has a low molecular weight, it eluted at the void volume of an AcA-54 (LKB) column with a molecular weight of greater than 80 kDa (Farrar et al, 1980 supra). In order to determine where PMA eluted following gel filtration normal medium containing 10 ng/ml PMA was concentrated 30-fold and chromatographed on the Sephadex G-100 column as described above. No CTL-inducing activity was associated with materials eluted at greater than 60kDa. Thus, the CTL-inducing activity of the 27 kDa factor was not likely due to residual PMA.

Further evidence that the 27 kDa factor is capable of inducing resting T-cells to respond to rIL-2 is found in Table 5 below.

Table 5. Effect of preculturing thymocytes in EL4.PMA or the 27kDa factor on their subsequent response to rIL-2.

| Balb/c thymocytes[a] | FACS analysis | | $^3$H-TdR Incorporation in response to | |
|---|---|---|---|---|
| | % BTCR+ | % IL-2R+ | 10 U/ml rIL-2 | 15% EL4.PMA |
| Fresh | 12.6 | 9.3 | $6,318 \pm 7698$ | $53,819 \pm 6123$ |
| Cultured for 2 days in 10 U/ml rIL-2 | 1.8 | 5.4 | $3,758 \pm 3063$ | $43,807 \pm 9421$ |
| Culture for 2 days in 15% EL4.PMA | 30.0 | 52.2 | $56,094 \pm 8112$ | $74,837 \pm 2595$ |
| Cultured for 2 days in 27kDa factor[b] | n.d. | 19.3 | $34,987 \pm 5475$ | $124,466 \pm 6012$ |

[a]The percentage of cells bearing the chain of the T-cell receptor (BCTR) or the IL-2 receptor (IL-2R) was determined using F23.1 or 7D4 mAb respectively in Balb/c thymocytes either directly or after culture for 2 days at $5 \times 10^5$ cells/ml in the presence of 10 U/ml rIL-2, 15% EL4.PMA. The proliferative response of Balb/c thymocytes to rIL-2 or EL4.PMA was determined directly or after culture for 2 days in rIL-2, EL4.PMA or the 27kDa factor.

[b]Fractions 39 to 48 from Fig. 1 were pooled and used at 15% (v/v). In addition to the CTL-inducing factor, this material had an IL-2 activity of 99 U/ml.

Fresh Balb/c thymocytes did not proliferate when stimulated with rIL-2. However, responsiveness to rIL-2 was induced following a 2-day incubation period with either crude EL4.PMA or the 27kDa factor (Table 5). Incubation of Balb/c thymocytes for 2 days in EL4.PMA or the 27kDa factor also led to a significant increase in the number of IL2R + cells (from 9.3 to 52.2 and 19.3%, respectively). The number of cells bearing the T-cell receptor chain also increased from 12.6 to 30.0% after culture in EL4.PMA. Taken together, these data suggest that the 27kDa factor in EL4.PMA probably acts by inducing the expression of IL-2 receptors on resting thymocyts. These IL-2 receptor positive cells can then be driven to proliferate in the presence of exogenously added rIL-2.

Example 7

In addition to their B-cell activating properties, IL-4 or B-cell stimulating factor (BSF-1) and IL-5 or T-cell replacing factor (TRF) also possess T-cell activating properties. rIL-4 can induce T-cell lines to proliferate and this response is inhibited by mAb to IL-4. IL-4, together with PMA, is also known to induce the production of cytotoxic cells from fetal thymocytes. However, this process appears from the literature to occur via an IL-2 independent mechanism since it was not inhibited by a mAb to the IL-2 receptor. Recombinant IL-5 is known to augment the proliferation of thymocytes in conjunction with suboptimal doses of ConA or IL-2. Since EL4.PMA is a source of IL-4 and IL-5, the ability of rIL-4 and rIL-5 to replace EL4.PMA in our assay system was investigated. As shown in Table 6, rIL-4 or rIL-5, with or without the addition of excess rIL-2, were unable to induce the production of cytotoxic cells. The CTL-inducing activity of EL4.PMA was also unaffected by the 11B11 Mab to IL-4 (lines 3 and 4). Furthermore, the addition of different concentrations of PMA (0.01 to 10 ng/ml) to cultures containing either rIL-4 or rIL-5 and rIL-2 did not increase the weak cytotoxic response observed when spleen cells were cultured with only rIL-2 and PMA.

Table 6

| B6 spleen cells | Culture Additions | | | | | % specific lysis | |
|---|---|---|---|---|---|---|---|
| | EL4.PMA % (v/v) | rIL-4 U/ml | rIL-5 5 (v/v) | rIL-2 U/ml | 11B11 g/ml | M | S.E. |
| $5 \times 10^3$ | | | | | | -1.1 ± | 2.0 |
| $5 \times 10^3$ | 10 | | | | | 46.6 ± | 10.0 |
| $5 \times 10^3$ | 10 | | | | 30 | 41.0 ± | 12.6 |
| $5 \times 10^3$ | 10 | | | | 10 | 43.4 ± | 13.4 |
| $5 \times 10^3$ | | 100 | | | | 2.1 ± | 1.7 |
| $5 \times 10^3$ | | 30 | | | | 1.3 ± | 0.7 |
| $5 \times 10^3$ | | 100 | | 20 | | 2.8 ± | 3.7 |
| $5 \times 10^3$ | | 30 | | 20 | | 1.8 ± | 1.6 |
| $5 \times 10^3$ | | | 10 | | | -0.3 ± | 1.3 |
| $5 \times 10^3$ | | | 5 | | | -0.8 ± | 0.6 |
| $5 \times 10^3$ | | | 10 | 20 | | -1.1 ± | 2.9 |
| $5 \times 10^3$ | | | 5 | 20 | | 1.4 ± | 1.3 |

[a]Culture and assay conditions are as described in Table 1.

[b]The activity of rIL-4 was determined as previously described by Mosmann et al, Proc. Nat'l. Acad. Sci. 83, 5654, 1986.

[c]rIL-5 was obtained by translation of a cloned cDNA in COS cells. The COS supernatant, when used at 10% (v/v), was able to induce the proliferation of $BCL_1$ cells; cmp over control = 51,985 for $1.5 \times 10^5$ cells/0.2 ml after a 2 day culture period. COS supernatant of the IL-5 cDNA clone, translated in the reverse direction, had no activity in the $BCL_1$ assay.

Thus, while generation of cytotoxic T-cells normally requires antigen and soluble lymphokines, it is clear that an alternative antigen-independent pathway exists in which the 27kDa functions, in combination with interleukin-2, to induce cytotoxic T-cell proliferation.

**Claims**

1. A soluble factor which participates in the antigen-independent activation of a cell-mediated immune response.

2. The soluble factor according to claim 1 characterised by being recoverable from extracelluar medium in which phorbol-12-myristate-13-acetate-stimulated EL4.IL-2 (ATCC TIB 181) cells have been cultured, and being capable of sensitising precursor cytotoxic T-cells to the proliferation-inducing effect of interleukin-2 in the absence of antigen or mitogen.

3. The soluble factor according to claim 2 further characterized by an approximate molecular weight of 27kDa.

4. A composition comprising a precursor cytotoxic T-cell sensitizing amount of the soluble factor defined in claim 2 or 3 and a cytotoxic T-cell proliferation-inducing amount of a compound having interleukin-2 activity.

5. A composition according to claim 4 which is substantially free of antigen or mitogen.

6. The composition defined in claim 4 or 5 wherein said compound having interleukin-2 activity is interleukin-2.

7. The composition according to any one of claims 4 to 6 which comprises a pharmaceutically acceptable carrier.

8. The composition according to any one of claims 4 to 7 in a form suitable for parenteral administration.

9. A method for inducing cytotoxic T-cell activity which comprises treating a population of precursor cytotoxic T-cells with the composition defined in any one of claims 4 to 8.

10. A composition as defined in claim 7 or 8 for use in a method of treatment of the human or animal body for restoring cytotoxic T-cell activity in a patient whose precursor cytotoxic T-cell population can be activated.

11. A process for producing the soluble factor defined in claim 2 which comprises culturing a population of cells which produce said soluble factor under conditions to stimulate the production thereof, fractionating medium in which said cells have been cultured to recover a fraction thereof comprising components having an approximate molecular weight of 27kDa and isolating said soluble factor from said fraction.

Figure 1